(19) **Europäisches Patentamt / European Patent Office / Office européen des brevets**

(11) **EP 2 638 798 B1**

(12) **EUROPEAN PATENT SPECIFICATION**

(45) Date of publication and mention of the grant of the patent:
**23.03.2016 Bulletin 2016/12**

(51) Int Cl.:
*A01H 4/00* *(2006.01)*      *C12N 5/04* *(2006.01)*
*C12M 1/00* *(2006.01)*      *C12P 19/44* *(2006.01)*
*C12P 19/60* *(2006.01)*     *C12P 7/42* *(2006.01)*
*C12P 17/06* *(2006.01)*

(21) Application number: **11805371.9**

(22) Date of filing: **09.11.2011**

(86) International application number:
**PCT/CL2011/000068**

(87) International publication number:
**WO 2012/061950 (18.05.2012 Gazette 2012/20)**

(54) **METHOD FOR THE CULTURE AND MASS MICROPROPAGATION OF DESCHAMPSIA ANTARCTICA IN VITRO**

VERFAHREN ZUR KULTIVIERUNG UND MASSENMIKROVERMEHRUNG VON ANTARKTISCHER SCHMIELE IN VITRO

PROCÉDÉ DE CULTURE ET DE MICROPROPAGATION MASSIVE IN VITRO DE DESCHAMPSIA ANTARCTICA.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2010 CL 12192010**

(43) Date of publication of application:
**18.09.2013 Bulletin 2013/38**

(73) Proprietor: **Universidad De Santiago De Chile**
**3363 Santiago (CL)**

(72) Inventors:
• **ZUÑIGA NAVARRO, Gustavo Emilio**
  **Santiago (CL)**
• **SEQUEIDA HONORATO, Alvaro Eduardo**
  **Santiago (CL)**
• **CASTRO OYARZUN, Alvaro Gonzalo**
  **Santiago (CL)**
• **TAPIA RODRIGUEZ, Eduardo Andres**
  **Santiago (CL)**
• **PRIETO ENCALADA, Humberto Godofredo**
  **Santiago (CL)**

• **ZAMORA CANTILLANA, Pablo Andres**
  **Santiago (CL)**

(74) Representative: **Carpintero Lopez, Francisco et al**
**Herrero & Asociados, S.L.**
**Alcalá 35**
**28014 Madrid (ES)**

(56) References cited:
**CN-A- 1 648 240      JP-A- 10 201 467**

• **XI-DA GU ET AL.: "UV-B Induced Changes in the Secondary Metabolites of Morus alba L. Leaves", MOLECULES, vol. 15, 27 April 2010 (2010-04-27), pages 2980-2993, XP002669864,**
• **CUBA ET AL.: "Micropropagation of Deschampsia antarctica - a frost-resistant Antartic plant", ANTARCTIC SCIENCE, vol. 17, no. 1, 2005, XP002669865, cited in the application**
• **ETIENNE H ET AL: "Temporary immersion systems in plant micropropagation", PLANT CELL, TISSUE AND ORGAN CULTURE, SPRINGER, NL, vol. 69, 1 January 2002 (2002-01-01), pages 215-231, XP002667315, ISSN: 0167-6857 cited in the application**

**Description**

The present invention refers to a method for *in vitro* culture and mass micropropagation of *Deschampsia antarctica* that allows to produce activated biomass in a multiplication time shorter than that known at present, and thereby, the biosynthesis of certain compounds useful to human health.

Background of the Prior Art

Of all the traditional products obtained by fermentation, secondary metabolites are the most important for human health. This product line includes antibiotics, certain toxins (mycotoxins), alkaloids (lysergic acid), plant growth factors (gibberellins), antioxidants and pigments. Thus, the products generated by plant secondary metabolism present a constant target to obtain products of economic interest. To obtain a medium- or high-scale productivity of the commercially attractive natural compounds, systems are required that: a) allow to develop important amounts of biomass of the organism of interest for a commercial exploitation of the same and b) make available designs that provide the appropriate conditions that will enable this organism to produce the metabolites that make it attractive.

The present invention combines these two productive concepts, describing the biomass multiplication and maintenance of the Antarctic resource *Deschampsia antarctica,* plant material, and the induction and exploitation of its properties as a natural source of active principles. The proposed system and the culture and stimulation method meet the requirements of basic and applied research, and may also be considered components of a system for producing a line of metabolites and natural extracts for commercial purposes, such as for the development of sunscreen lotions.

The importance of the antioxidant and photo protective compounds synthesized by *D. antarctica* raises the technical challenge of developing improved systems that allow to increase their concentration in the plants used as a source, under controlled operational (Or experimental) conditions appropriate for their synthesis. The present photo-thermal bioreactor and method allows making a high-scale use of this resource. As an end product, the use of scaling-up systems allows to propose the isolation and purification of compounds having characteristics that may be used, for example, in human health, as food supplements, sunscreens and cosmetics.

The use of bioreactors in plants is made available as an essential tool for an automatic production in an enhanced volume of various plant species. Times and production costs may be reduced with their use, and optimizations in the multiplication kinetics of the plant material may be characterized and proposed in strategic locations such as in metabolite production of a growth-associated, partially-growth associated and non-growth associated nature. There are a great number of bioreactor designs for plant cell culture (for example, Afreen, F. (2006) Temporary Immersion Bioreactor: Engineering considerations and applications in plant micropropagation. Focus on Biotechnology-Ptant Tissue Culture Engineering, pp 187-200. Springer Dordrecht, The Netherlands; Aitken-Christie, J., Singh A., Horgan, K., Thorpe, T. (1985). Explant Developmental State and Shoot Formation in Pinus radiata Cotyledons. Botanical Gazette 146: 196-203; Banerjee, S. (1999). In vitro multiplication of Centella asiatica, a medicinal herb from leaf explants. XP 000937832; Cuba, M., Gutierrez-Moraga, A., Butendiek, B. and Gidekel, M. (2005). Micropropagation of Deschampsia antarctica - a frost resistant Antarctic plant. Antarctic Sci. 17-69-70; Endress, T. (1994). Plant Cell Biotechnology Springer-Verlag, pp 121-246; Etienne H. and Berthouly M. (2002). Temporary immersion systems in plant micropropagation. Plant Cell, Tissue and Organ Culture 69:215-231; Etienne, H., Dechamp, E., Barry-Etienne, D. and Bertrand, B. (2006). Bioreactors in coffee micropropagation. Brazilian Journal of Plant Physiology 18:45-54; McAlister, B., Finnie, J., Watt, M. P. and Blakeway, F. (2005). Use of the temporary immersion bioreactor system (RITA) for production of commercial Eucalyptus clones in Mondi Forests (SA). Plant Cell, Tissue and Organ Culture 81:347-358; Paek K., Hahn E. and Son S. (2001). "Application of bioreactors for large-scale micro propagation system of plants". In vitro Cell. Dev. Biol. - Plant 37:149-157; Quiala, E., Barbón, R., Jiménez, E., De Feria, M., Chávez, M., Capote, A., and Pérez, N. (2006). Biomass production of Cymbopogon citratus (DC) Stapf., a medicinal plant, in temporary immersion systems. In Vitro Cellular & Developmental Biology 42:298-300; Roels S., Noceda C., Escalona M., Sandoval J., Canal M.J., Rodriguez R. and Debergh P. (2006). The effect of headspace renewal in a Temporary Immersion Bioreactor of plantain (Musa AAB) shoot proliferation and quality. Plant Cell, Tissue and Organ Culture 84:155-163; Zhu L., Li X. and Welander M. (2005). Optimization of growing conditions for the apple rootstock M26 grown in RITA containers using temporary immersion principle. Plant Cell, Tissue and Organ Culture 81:313-318), wherein temporary immersion systems stand out for their ease of handling.

These design types have clear advantages over conventional micropropagation platforms and are of a great plasticity to modify their design in order to increase the concentration of the metabolites of interest together with biomass increase, saving work hours and minimizing as well costs associated with the process.

Temporal immersion systems are defined as methods for temporary soaking a tissue culture by immersing it into a liquid nutrient, followed by drainage of the medium. The system operates with immersion and drainage cycles, and the period of immersion, as well as the frequency at which said immersion occurs are decisive factors for the culture, these cycles depending on the requirements and nature of the tissue being cultured. *In* some cases immersions may be periodical, or comprise from four to six immersions per day (Endress, R. (1994). Plant Cell Biotechnology Springer-Verlag, pp 121-246; Etienne H. and Berthouiy. M. (2002). Temporary immersion systems in plant micropropagation. Plant Cell, Tissue and Organ Culture 69:215-231). Temporary immersion is a new culture classification that combines the advantages presented by the solid medium (maximum gas exchange) and the liquid medium (increased nutrient absorption) Etienne H. and Berthouly M. (2002). Temporary immersion systems in plant micro propagation. Plant Cell, Tissue and Organ Culture 69:215-231), which allows to avoid the problems frequently occurring in a permanent culture, such as asphyxia (in the absence of adequate stirring and aeration) and hyperhydration that results in a series of physiologic disorders in the tissue that create an appearance of vitrification. (Etienne, H., Dechamp, E., Barry-Etienne, D. and Bertrand, B. (2006). Bioreactors in coffee micropropagation. Brazilian Journal of Plant Physiology 18:45-54).

In order to reduce costs and further facilitate management of temporary immersion bioreactors, designs have constantly been modified until obtaining designs where the culture medium is pneumatically transferred to the chamber containing the tissue culture (Afreen, F. (2006), Temporary Immersion Bioreactor: Engineering considerations and applications in plant micropropagation. Focus on Biotechnology - Plant Tissue Culture Engineering, pp 187-200; and Roels S., Noceda C., Escalona M., Saridoval J., Canal M.J., Rodriguez R. and Debergh P. (2006). The effect of headspace renewal in a Temporary Immersion Bioreactor of plantain (Musa AAB) shoot proliferation and quality. Plant Cell, Tissue and Organ Culture 84:155-163). Bioreactors RITA® and BIT® are within the established designs in the market. This system entirely eliminates *in vitro* subculture stages arid all the characteristic steps conducted for seedling generation are performed in the same chamber, not requiring moving the tissue, only to modify the culture medium (Paek K., Hahn E. and Son S. (2001). "Application of bioreactors for large scale micropropagation system of plants". In vitro Cell. Dev. Biol. - Plant 37:149-157) with all the advantages this offers, such as time optimization in the successive subcultures of conventional micropropagation as well as cost and contamination hazard reduction.

The most important characteristics of a temporary immersion bioreactor are (Afreen, F. (2006), Temporary Immersion Bioreactor: Engineering considerations and applications in plant micropropagation. Focus on Biotechnology - Plant Tissue Culture Engineering, pp 187-200):

- Hyperhydration reduction: compared to that present in permanent immersions, this is a significant achievement. As the plants are immersed in the liquid culture medium only for a few minutes every 3-6 hours, physiologic disorders are reduced and plants become healthier.
- Plant growth and development may be controlled by managing the frequency and duration of immersions in the liquid medium.
- Plant growth is improved, because in each immersion the plant is in direct contact with the culture medium and a thin coat of the liquid that covers the tissue is formed in the time interval during immersions.
- Air vents in the culture chamber prevent contamination of the same.
- Due to the absence of stirring or aeration, mechanical stress on the plant material is much lower compared to other bioreactor systems.

However, to this date there isn't any culture system that includes micro environmental variables such as the temperature, luminescence, radiation and gas interchange required by *Deschampsia antarctica* to generate the compounds of interest.

1. Reactors in *Deschampsia antarctica* culture/ production

A great number of experiences have been reported describing the operation of this type of systems with different plant species. Temporary immersion has been used in the propagation of commercial ligneous species such as pine and eucalyptus (Aitken-Christie, J., Singh A., Horgan, K., Thorpe, and T. (1985). Explant Developmental State and Shoot Formation in Pinus radiata Cotyledons. Botanical Gazette 146: 196-203; McAlister, B., Finnie, J., Watt, M. P. and Blakeway, F. (2005). Use of the temporary immersion bioreactor system (RITA) for production of commercial Eucalyptus clones in Mondi Forests (SA). Plant Cell, Tissue and Organ Culture 81:347-358), fruit species such as apple, banana and pineapple (Zhu L., Li X. and Welander M. (2005). Optimization of growing conditions for the apple rootstock M26 grown in RITA containers using temporary immersion principle. Plant Cell, Tissue and Organ Culture 81:313-318; Roels S., Noceda C., Escalona M., Sandoval J., Canal M.J., Rodriguez R. and Debergh P. (2006). The effect of headspace renewal in a Temporary Immersion. Bioreactor of plantain (Musa AAB) shoot proliferation and quality. Plant Cell, Tissue and Organ Culture 84:155-163) as well as coffee (Etienne, H., Dechamp, E., Barry-Etienne, D. and Bertrand, B. (2006). Bioreactors in coffee micropropagation. Brazilian Journal of Plant Physiology 18:45-54), among many others. The initial plant material may also differ in its initial developmental stage that very often starts from somatic embryos, as well as from plant material, shoots, and even using adventitious root cultures. The above-referred publications report similar systems known for other species, other designs and conducting other strategies.

The use of temporary immersion systems has been employed in the propagation of species to obtain secondary metabolites that may be used to elaborate products that are beneficial to human health, as in the case of lemon grass (Quiala, E., Barbón, R., Jiménez, E., De Feria, M., Chávez, M., Capote, A., and Pérez, N. (2006). Biomass production of Cymbopogon citratus (DC) Stapf., a medicinal plant, in temporary immersion systems. In Vitro Cellular & Developmental Biology 42:298-300). The same has occurred in documents that claim the property of systems for exploiting medicinal plants such as *Hypericum perforatum, Scutellaria baicalensis and Allium* sp. (Roels S., Noceda C., Escalona M., Sandoval J., Canal M.J., Rodriguez R. and Debergh P. (2006). The effect of headspace renewal in a Temporary Immersion Bioreactor of plantain (Musa AAB) shoot proliferation and quality. Plant Cell, Tissue and Organ Culture 84:155-163) including *Centella asiatica* (Banerjee, S. (1999). In vitro multiplication of Centella asiatica, a medicinal herb from leaf explants, (XP 000937832), that are commercially used as antidepressants, antibacterials, antiseptics and cicatrizants, respectively.

Specifically, micropropagation of *D. antarctica* has rarely been described and then only through the use of solid cultures (Cuba, M., Gutierrez-Moraga, A., Butendiek, B. and Gidekel, M. (2005). Micropropagation of Deschampsia antarctica - a frost-resistant Antarctic plant. Antarctic Sci. 17:69-70). In this publication, the same authors indicate that said system is capable of stimulating multiplication only after 15 days of initiating the culture with a system based on a solid culture. In the present system and method, biomass is doubled 14 days after culture initiation. There are no other documents in the state of the art that refer to the artificial maintenance or micropropagation of this species.

On the other hand, the present photo-thermal bioreactor and method is useful to induce in this species metabolites that can be used in various activities of human interest. The present photo-thermal bioreactor and method allows to replace culture media entirely or to provide pulses with inducing agents to elicit a response from the plant. In the state of the art there are some systems that claim photo bioreactors for photosynthetic species in general (Banerjee, S. (1999). In vitro multiplication of Centella asiatica, a medicinal herb from leaf explants. XP 000937832; Endress, R. (1994). Plant Cell Biotechnology Springer-Verlag, pp 121-246. However, the present photo thermo-bioreactor is a single unit, intended for metabolite propagation, maintenance and induction, inducing their production in *D. Antarctica*.

Objects of the Invention

The object of the present invention is a method for efficient *in vitro* culture and mass micropropagation of the antarctic resource *Deschampsia antarctica* by using temporary immersion, wherein secondary metabolites having antioxidant and/or photo protective properties useful to human health may be induced.

Specific Objects of the Invention

A specific object of the invention is the development of a method for the culture, micropropagation, maintenance and biologic induction of *D. antarctica* secondary metabolites.

Description of Figures

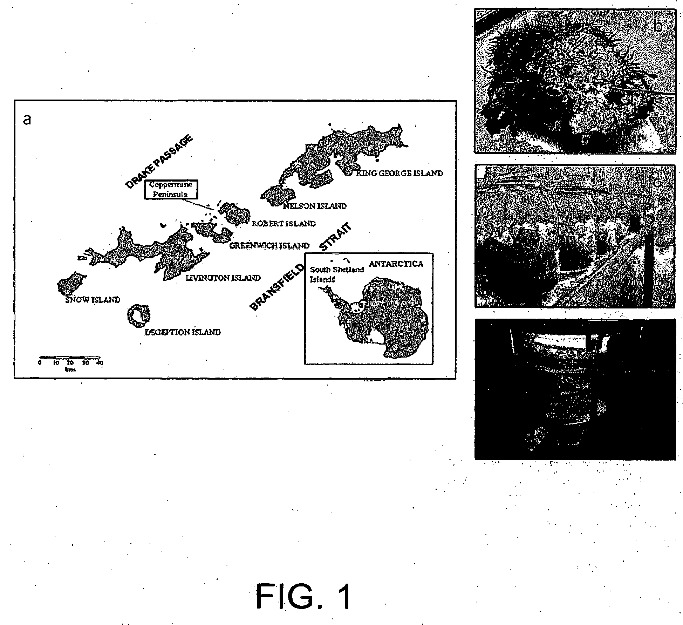

Figure 1. Plants recovered from their sampling area and introduced into an *in vitro* culture to be subsequently used for metabolite mass production and induction. (a) shows the geographic area of their extraction: Robert Islands, South Shetland Islands, Maritime Antarctic (64° 24'S; 59° 30'W) in the year 2004; (b) shows the conditions in which *Deschampsia antarcfica* plants were collected; (c) shows the introduction of the plant material into an *in vitro* culture; (d) shows the photo-thermal bioreactor for mass production of biomass with secondary metabolite induction by ultraviolet light B (UV-B).

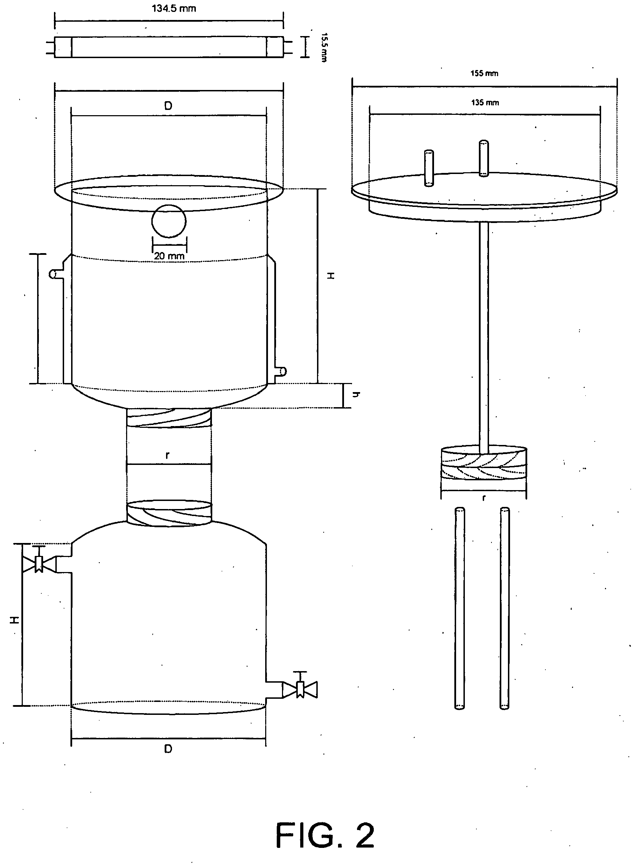

Figure 2. Design of the glass chambers (left) and stainless steel parts (right) for the temporary immersion photo-bioreactor that incorporates ultraviolet light B.

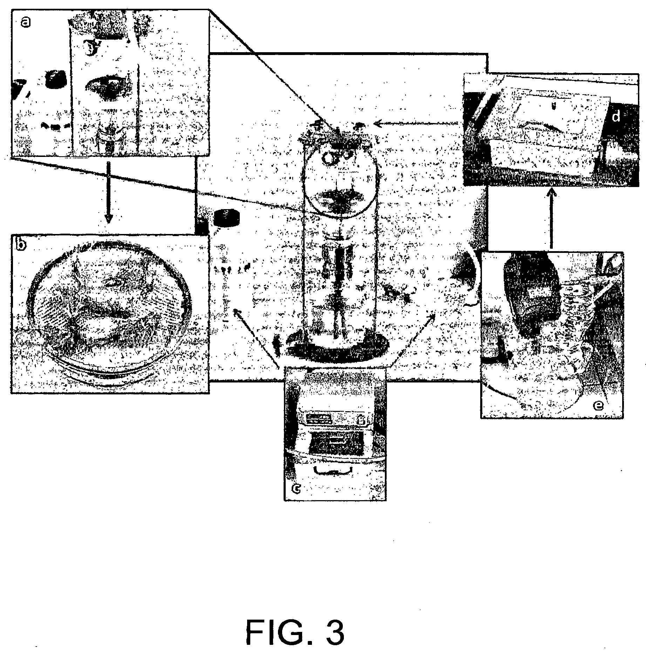

Figure 3. General view of the photo-thermal bioreactor for multiplication. A glass jacket reproduces climatic conditions in the growth container (Figure 3a). Temperature is regulated by a precision thermoregulating bath (Figure 3c). The plant material is supported by a stainless steel screen (Figure 3b). The lower chamber that holds the culture medium comprises inlet and outlet valves. Immersions take place due to the pneumatic action of a pressure pump (Figure 3e). A timer experimentally determines and controls flows for these immersions. Figure 3d).

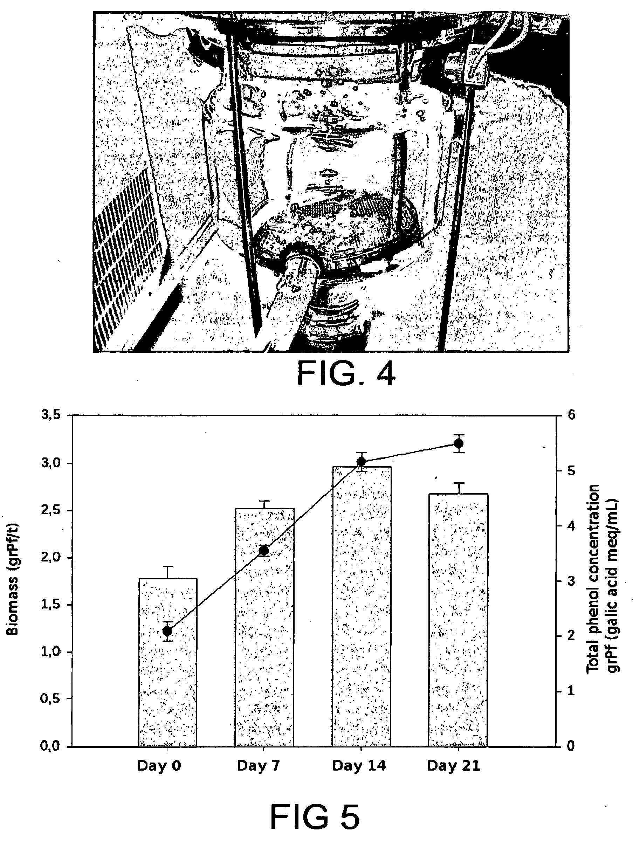

Figure 4. Lighting means stimulate secondary metabolite synthesis in the biomass by means of UV-B light. The total length of ultraviolet-B light tube is 13-45cm (Tec/West USA Inc, Los Angeles, CA, USA).

Figure 5. Biomass concentration and total phenolic compounds in *Deschampsia anfarctica* plants grown in the photo-thermal bioreactor. It shows biomass concentration (bars) and total phenolic concentration during growth kinetics. gPf: grams of fresh weight.

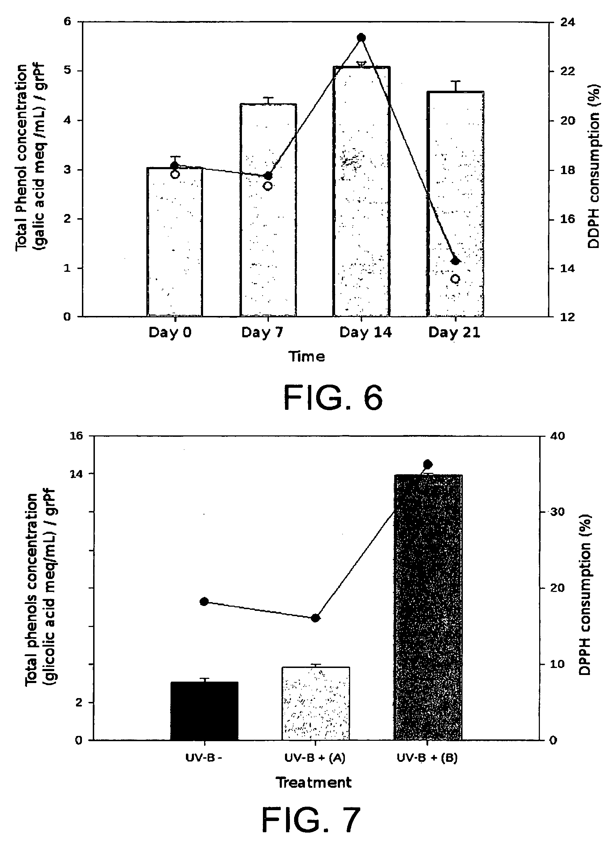

Figure 6. Total phenolic compounds concentration and antioxidant biologic activity in *Deschampsia antarctica* plants grown in the photo-thermal bioreactor. It illustrates a comparison of total phenolic concentration (bars) and trapping capacity of free radical 2,2-diphenyl-1-picrylhydrazyl DDPH, (line) over the kinetics of biomass multiplication.

Figure 7. Effect of the application of UV-B radiation on the concentration of total phenolic compounds and the trapping capacity of free radicals on *Deschampsia antarctica* plants grown in the photo-thermal bioreactor. It shows total phenolic concentration (bars) and free radical DPPH (line) consumption capacity; UV-B, plants with no UV-B exposure; UV-B + (A), Plants exposed to UV-B for 4 continuous hours per day; UV-B + (B), Plants exposed to UV-B for 30 min at 6-hour intervals per day. All samples were taken by triplicate on seedlings grown for 7 days. gPf: grams of fresh weight. DDPH: 1,1-diphenyl-2-picryl-hydrazyl.

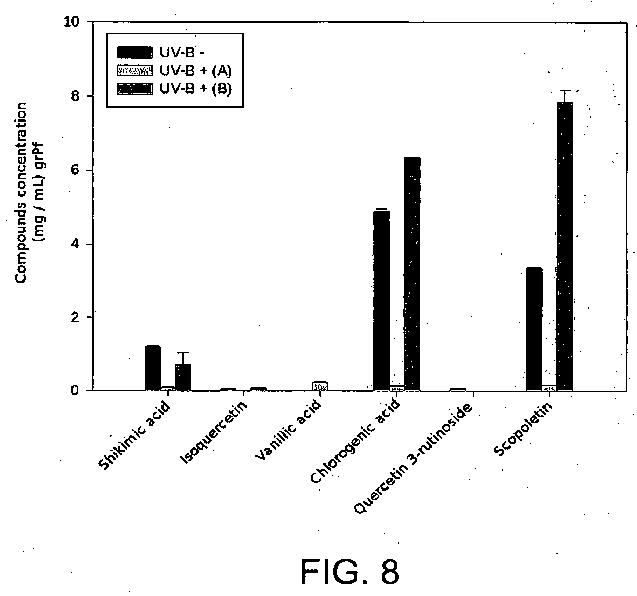

Figure 8. Concentration of different phenol compounds in *Deschampsia antarctica* plants grown in the photo-thermo reactor. Concentrations of shikimic acid, isoquercetin, vanillic acid, chlorogenic acid, quercetin 3-rutinoside and scopoletin are shown as determined by HPLC-DAD. UV-B-plants with no UV-B exposure; UV-B + (A), Plants exposed to UV-B for 4 continuous hours per day; UV-B + (B), Plants exposed to UV-B for 30min at 6-hour intervals per day. All determinations were made on seedlings grown for 7-21 days in the photo-thermal bioreactor.

## Description of the Invention

*Deschampsia antarctica* Desv. is one of the two native vascular plants in Maritime Antarctica, habitat that is simultaneously affected by various extreme environmental factors such as high UV-B levels, low temperatures and low water availability. It has been reported that *D. antarctica* systems are highly efficient to tolerate these extreme environmental factors, exhibiting at the same time a high photosynthetic rate. These properties make this plant produce a great endogenous accumulation of compounds derived from its secondary metabolism, their function being that of acting as photo protectors, cryoprotectors, osmoprotectors and sugars in general. In this respect, this resource of the vascular Antarctic flora is a natural source of compounds having an evident photoprotective and antioxidant function; it is necessary to add to this the existence of still unexplored genes and regulating sequences of great interest, together with their gene products (proteins having known and unknown functions).

A system for *in vitro* biomass multiplication of this species is described, with technical requirements that are evidently those that best simulate the conditions of its natural habitat. In this sense a nutrient system has been developed to allow biomass maintenance, growth and multiplication, In addition, these nutrients are used to feed the plant material in an automated temporary immersion system in order to provide the exact nutrient dosage. Furthermore, the system contemplates the use of a temperature and a light source that more accurately simulate the plant environmental conditions; to this end, the reactor model contemplates reproducing its climatic conditions by reducing temperature with cooling means, preferably a thermally regulated bath, and more specifically, lighting the growing plant material by means of light sources that are preferably, but not limited to UV-B radiation.

## Detailed Description of the Invention

1. Collection of the parent material, explorations and establishment into *in vitro* cultures.

Samples of D. *antarctica* were collected during explorations conducted in Robert Island (South Shetland Islands, Maritime Antarctica) in the year 2004. The collected samples were taken to the laboratory and recovered by introducing them into an *in vitro* culture system, using for this purpose nutrients for their maintenance and micropropagation.

2. Transference of the recovered plant material to the scaled-up production system.

The recovered material is introduced into a culture system based on the use of a semiautomatic photo-thermal bioreactor for temporary immersion specially designed for the species. The photo-thermal bioreactor has been designed to comprise a double chamber with a lower compartment for nutrient storage and an upper compartment for biomass development. An immersion in nutrients is used to maintain and increase the biomass, this immersion being further supplemented by conditions of temperature and light specifically suitable for the species being propagated. The systems of thermal regulation and radiation generation are a structural part of the upper compartment for the above described culture system, thus making the design specific and unique. The optimum temperature conditions for growth and lighting/radiation have been adjusted to the growth conditions recorded in the plant material habitat. Immersion flows of the plant material are also time regulated by means selected from clocks, valves and air pumps.

3. Biomass and secondary metabolite induction in the phono-thermal bioreactor.

Due to the improved nutrient and gas transference between plant materials and nutrients, biomass production is doubled in the photo-thermal bioreactor after a 7-day culture period. Likewise, the production of antioxidant compounds in the plant material cultured in this system increases in this same culture period, being further over-stimulated by the application of UV-B light pulses during culture thereof.

EXAMPLES

**Example 1**: Introduction of *Deschampsia antartica* samples obtained *in situ* for *in vitro* recovery thereof

The initiation and recovery media used to introduce Antarctic samples of D. *antarctica* in containers with nutrients (Figure 1) are indicated in Table 1.

**Table 1.** Compositions of the culture media for initiation, recovery and maintenance in *Deschampsia Antarctica* plants micropropagation

| Composition | Concentration |
| --- | --- |
| MS Basal Medium (Phyto Tech Lab™) | 4.43 g/L |
| Saccharose | 20 g/L |
| Kinetin | 0.2 mg/mL |
| Benzylaminopurine (BAP) | 0.3 mg/mL |
| Biotin | 0.1 mg/mL |
| pH 5.6-5.7 | |

| Composition | Concentration |
| --- | --- |
| MS Basal Medium (Phyto Tech Lab™) | 4,43 g/L |
| Saccharose | 30 g/L |
| Isopentenyl adenine (2IP) | 0.55 mg/mL |
| pH 5.6-5.7 | |

| Composition | Concentration |
| --- | --- |
| MS Basal Medium (Phyto Tech Lab ™) | 4,43 g/L |
| Saccharose | 20 g/L |
| Indole Butyric acid (IBA) | 0.01 mg/mL |
| Gibberellic acid (GA3) | 0.1 mg/mL |
| pH 5.6-5.7 | |

| Composition | Concentration |
| --- | --- |
| MS Basal Medium (Phyto Tech Lab ™) | 4.43 g/L |
| Saccharose | 20 g/L |
| IBA | 0.01 mg/mL |
| BAP pH 5.6-5.7 | 0.3 mg/mL |

| Composition | Concentration |
| --- | --- |
| MS Basal Medium (Phyto Tech Lab™) | 4,43 g/L |
| Saccharose | 20 g/L |
| IBA | 0.05 mg/mL |
| BAP | 0.3 mg/mL |
| pH 5.6-5.7 | |

| | |
| --- | --- |
| MS Basal Medium (Phyto Tech Lab™) | 4.43 g/L |
| Saccharose | 20 g/L |
| BAP pH 5.6-5.7 | 0.3 mg/mL |

| Composition | Concentration |
| --- | --- |
| MS Basal Medium (Phyto Tech Lab™) | 4.43 g/L |
| Saccharose | 20 g/L |
| Kinetin pH 5.6-5.7 | 0.02 mg/mL |

The composition of the culture medium described by Murashige and Skoog (MS medium) is widely known in the state of the art since 1962 (Murashige T., Skoog F. 1962. A revised medium for rapid growth and bioassays with tobacco tissue cultures. Physiol. Plant 15,473-497).

Figure 1 shows the entire method for collection of the species *Deschampsia antartica* and its introduction into *in vitro* cultures for recovery thereof. Figure 1 a details the geographic site of their collection: Robert Island, South Shetland Islands, Maritime Antarctic (64° 24'S; 59° 30'W) in 2004; a detail of the collected sample type is shown in Figure 1b. The final conditions of *D. antarctica* recovery stage for an *in vitro* culture are shown in Figure 1c. Finally, Figure 1d shows the stage of propagation or mass multiplication and induction described in the following Examples.

**Example 2.** *Ad hoc* Immersion System Design.

There are no established records of geometric configurations describing the design of a temporary immersion bioreactor (different from the air-lift, bubble column. etc. types of bioreactors), so that the equipment dimensions must be based on the particular plant culture requirements. Temporary immersion bioreactors basically require for their operation equipment similar to that used in air-lift and bubble column bioreactors, with the difference that the plant material that is going to be propagated in temporary immersion bioreactors is held on a support that may be stationary or floating. The design of a temporary immersion bioreactor generally comprises two chambers that are independent from one another, one that contains the culture medium and the other that holds the plant material that is being cultured. The culture medium is pneumatically transferred from one chamber to the other due to the air overpressure exerted on the surface of the culture medium, transference starting from one chamber to the other; this transference also renews the atmosphere of the chamber containing the plant material and causes a slight stirring and oxygenation of the medium (Roels S., Noceda C., Escalona M., Sandoval J., Canal M.J., Rodriguez R. and Debergh P. (2006). The effect of headspace renewal in a Temporary Immersion Bioreactor of plantain (Musa AAB) shoot proliferation and quality. Plant Cell, Tissue and Organ Culture 84:155-163). When aeration is discontinued, the culture medium returns to its original chamber. A solenoid valve controlled by a common irrigation programmer regulates air injection from the air compressor.

Some simple expressions that yield the cylinder and truncated cone volumes may be used for its design; to this end, the cylinder volume is obtained as follows:

$$V_c = \pi H_c \left( \frac{D_D}{2} \right)^2 \qquad \text{(Eq. 1)}$$

wherein height $H_c$ is assumed as the maximum height attained by D. *antarctica* in an *in vitro* culture, which is about eight centimeters before proceeding to plant transplanting.

The truncated cone volume is obtained from the following expression:

$$V_t = \left( \frac{\pi \cdot h}{3} \right) \left( D_D{}^2 + r^2 + D_D r \right) \qquad \text{(Eq. 2)}$$

and the total volume that comprises both the cylinder and the truncated cone is:

$$V_T = V_c + V_t \qquad \text{(Eq. 3)}$$

The total volume of the truncated cone section is not included in the lower chamber dimensions, because the volume of said section will be used as the space that will exert overpressure on the culture medium surface; thus only the expression of the cylinder volume of this chamber must be contained in the operating volume of the upper chamber that includes the truncated cone section of the same, using only equation Eq.1.

Now, liquid height must not encompass the entire dimensions of the upper chamber so as to leave a proper space for gas interchange and prevent immersion of the UV-B light tube in the culture medium; in the present invention the upper chamber height has been defined to have an over dimension using the operating volume ratio = 0,6 x (total volume). Figure 2 shows the final design of the automated liquid immersion system for the resulting *D. antarctica* culture and Table 2 in internal lighting or luminescence (UV-B light). means that allow the induction of UV-inducible compounds by this multiplying biomass.

**Table 2:** Summary of the temporary immersion bioreactor dimensions

| Section Upper chamber | Length (cm) | Volume (mL) |
|---|---|---|
| Maximum height D.a may Attain in the system (Hc) | 80 (x 1.5) | X |
| Section diameter (D) | 115 | X |
| Radius (R) | 50 | X |
| Height (H) | 30 | X |
| Upper Cont. Vol.* | X | 1,504.5 |
| **Lower chamber** | | |
| Lower chamber height (Hi) | 120 | X |
| Lower Rec. Vol. | X | 1,245.8 |
| Cone volume (Vc) | X | 830.5 |
| Trunk volume (Vt) | X | 674.3 |

*: The upper chamber volume results from adding Vc and Vt without considering its height oversize, a value considered in the lower chamber design.

1. Figure 3 exhibits details of the multiplication photo-thermal bioreactor. The growth chamber reproduces climatic conditions by using a glass jacket (Figure 3a) that allows biomass multiplication at optimum temperatures for *D. antarctica.* Temperature is regulated by a thermoregulating precision bath (Figure 3c). The plant material is supported on a screen made of stainless steel (Figure 3b) or of inert ceramics or inert plant material. The lower chamber that contains the culture medium is provided with inlet and outlet valves, the purpose of said valves is to exchange fresh culture medium, offering as well the advantage of optionally adding a compound ("elicitor" or inducing agent) to activate a metabolic path of interest, to provide hormonal pulses to an immersion depending on the culture growth stage, or simply to take samples of the kinetics of the consumption of nutrient or culture means by the tissue being cultured. Immersions are carried out by the pneumatic action of a pressure pump (Figure 3e). The flows for these immersions are experimentally determined and controlled by a timer (Figure 3d).

An additional aspect of the photo-bioreactor for *D. anfarctica* culture is the addition of a biomass stimulation system using UV energy, by internal lighting or luminescent means. The system has been designed as shown in Figure 4.

Example 3. Biomass Duplication

Biomass development in the photo-thermal bioreactor is shown in Figure 5. An initial inoculation of 1,8g of *D. antarctica* shoots obtained after the recovery culture (Example 1) was made by depositing them on the reactor support system, and biomass production was recorded by wet weight determination. Basal MS was used as a culture medium for mass production (Table 1, Example 1) supplemented with saccharose 2%w/v, kinetin 0.2mg/ml, BAP 0.3mg/ml and biotin 0.1 mg/ml. The pH of the culture medium was 5.6 - 5.7.

**Example 4.** Kinetics of Total phenolic Induction and Antioxidant Biologic Activity.

The synthesis of secondary metabolites is one of the main points of interest in the present invention. An assessment of the total phenolic compounds and the antioxidant biologic activity in *Deschampsia anfarctica* plants grown in the photo-thermal bioreactor is illustrated for the above-illustrated kinetics of biomass multiplication. Figure 6 shows total phenolic concentration (bars) and DPPH free radical trapping capacity (illustrated by lines) during the evaluated culture period.

**Example 5.** Total phenolic Induction and Antioxidant Biologic Activity Using UV Radiation.

The effect of the application of UV-B radiation by the internal lighting system of the photo-thermal bioreactor on the concentration of total phenolic compounds and the trapping capacity of free radicals in *Deschampsia antarctica* plants grown therein is illustrated in Figures 7 and 8.

To obtain the induction effect on the biomass culture, UV light pulses were applied for a 7-day culture period, determining thereafter the total concentration of phenol compounds (bars) and the consumption capacity of DPPH free radical (line), as indicative of the antioxidant capacity. Two lighting treatments were applied; the use of a 4-hour UV pulse/day over a 7-day culture showed that it did not induce more compounds of this type compared to a control not subjected to UV pulse. However, when a 30-minute UV pulse was applied every 6 hours over this same culture period, the phenol compounds and antioxidant activity levels were much higher than the control not subjected to lighting (Figure 7). It was found that within the compounds identified as induced by this UV light treatment there is an important accumulation of scopoletin, and to a lesser extent, of chlorogenic acid, quercetin and rutin as the result of the brief application of UV, compared to a control that was not subjected to this radiation (Figure 8).

**Claims**

1. A method for an *in vitro* culture and mass micropropagation of *Deschampsia antarctica (D. antarctica)* plant material, further inducing generation or biosynthesis of antioxidant and photoprotective metabolites characteristic of said plant material, such as scopoletin, chlorogenic acid, rutin, and quercetin, comprising the following steps:

   a) collecting parent plant material consisting of *Deschampsia antarctica;*
   b) recovering said plant material from the collected parent material;
   c) inoculating said plant material shoots;
   d) *in vitro* culturing and micropropagating said plant material by temporary immersion in MS Basal Medium (*PhytoTech* Lab™) that further comprises sugars, hormones, vitamins and cytokines and is at a pH between 5-6, under temperature conditions that simulate those of *D. antarctica* natural habitat;
   e) inducing the plant material of secondary compounds production by applying UV-B lighting pulses,

   wherein the following are used in step d): sugars selected from saccharose, glucose or fructose; hormones selected from benzyl amino purine (BAP), isopentenyl adenine (2IP), indole butyric acid (IBA), GA3 or a mixture thereof; vitamins selected from biotin and citokines;
   further, in step d), a specific nutrient dose is supplied to the plant material, controlling the plant material immersion time in the nutrients or in the culture medium; and
   step e) comprises the application of 30-minute UV-B lighting every 6hours for a culture period of 7-21days.

2. A method for an *in vitro* culture and micropropagation of *D. antarctica* according to claim 1, wherein after step e) it further comprises:

   f) determining the concentration of total phenolics produced by the plant material after step e), and determining the oxidizing capacity of the same; and/or
   g) phenol extraction and chemical characterization thereof.

3. A method for an *in vitro* culture and micropropagation of *D. antarctica* according to claim 1, wherein step d) is conducted in a culture medium comprising 1-3% saccharose.

4. A method for an *in vitro* culture and micropropagation of *D. antarctica* according to claim 1, wherein step d) is conducted in a culture medium, 0.2mg/ml kinetin.

5. A method for an *in vitro* culture and micropropagation of *D. antarctica* according to claim 1, wherein step d) is conducted in a culture medium comprising 0.3mg/ml, BAP.

6. A method for an *in vitro* culture and micropropagation of *D. antarctica* according to claim 1, wherein step d) is conducted in a culture medium comprising 0.1mg/ml biotin.

7. A method for an *in vitro* culture and micropropagation of *D. antarctica* according to claim 1, wherein step d) comprises culturing and micropropagating the plant material into a culture medium comprising 2 and 5g/L MS Basal Medium (*Phyto* Tech Lab™), 20g/L saccharose, 0.2mg/mL kinetin, 0.3mg/mL BAP, 0.1mg/mL biotin, 0.01mg/mL IBA and 0.01mg/mL GA3.

8. A method for an *in vitro* culture and micropropagation of D. *antarctica* according to claim 1, wherein step d) comprises culturing and micropropagating the plant material into a culture medium comprising 2-5g/L MS Basal Medium (*PhytoTech* Lab™), 10-30g/L saccharose, 0.1-0.55mg/mL 2IP.

9. A method for an *in vitro* culture and micropropagation of *D. antarctica* according to claim 1, wherein step d) comprises culturing and micropropagating the plant material into a culture medium comprising 4.4g/L MS Basal Medium (*PhytoTech* Lab™), 20g/L saccharose, 0.01mg/mL IBA and 0.01mg/mL GA3.

10. A method for an *in vitro* culture and micropropagation of *D. antarctica* according to claim 1, wherein step d) comprises culturing and micropropagating the plant material into a culture medium comprising 4.43g/L MS Basal Medium (PhytoTech Lab™), 20g/L saccharose, 0.01mg/mL IBA, 0.3mg/mL BAP and at pH between 5.6-5.7.

11. A method for an *in vitro* culture and micropropagation of *D. antarctica* according to claim 1, wherein step d) comprises culturing and micropropagating the plant material into a culture medium comprising 4.43g/L MS Basal Medium (PhytoTech Lab™), 20g/L saccharose, 0.05mg/mL IBA and 0.3mg/mL BAP and at pH between 5.6-5.7.

12. A method for an *in vitro* culture and micropropagation of *D. antarctica* according to claim 1, wherein step d) comprises culturing and micropropagating the plant material into a culture medium comprising 4.43g/L MS Basal Medium (*PhytoTech* Lab™), 20g/L saccharose, 0.3mg/mL BAP and at pH between 5.6-5.7.

13. A method for an *in vitro* culture and micropropagating of *D. antarctica* according to claim 1, wherein step d) comprises culturing and micropropagating the plant material into a culture medium comprising 4.43g/L MS Basal Medium (*PhytoTech* Lab™), 20g/L saccharose, 0.02mg/mL BAP.

14. A method for an *in vitro* culture and micropropagation of *D. antarctica* according to claim 1, wherein it further comprises step d') of adding by means of pulses, chemical inducing agents selected from salts, metals, organic compounds such as hormones and the like.

15. A method for an *in vitro* culture and micropropagation of *D. antarctica* according to claim 1, wherein it further comprises step d") of taking samples of the consumption of nutrient or culture medium by the plant material.

**Patentansprüche**

1. Verfahren für eine *In-vitro-Kultur* und Massenmikrovermehrung von *Deschampsia antarctica (D.* antarctica)-Pflanzenmaterial, wobei das Verfahren des Weiteren die Erzeugung oder Biosynthese von antioxidativen und photoprotektiven Stoffwechselprodukten induziert, die für dieses Pflanzenmaterial charakteristisch sind, wie beispielsweise Scopoletin, Chlorogensäure, Rutin und Quercetin, umfassend die folgende Schritte:

   a) Sammeln von Ausgangspflanzenmaterial bestehend aus *Deschampsia antarctica*;
   b) Gewinnen des Pflanzenmaterials aus dem gesammelten Ausgangsmaterial;
   c) Beimpfen der Sprossen des Pflanzenmaterials;
   d) *In vitro*-Kultivieren und Mikrovermehren des Pflanzenmaterials durch temporäre Immersion in MS Basal Medium (*Phyto*Tech Lab™), das des Weiteren Zucker, Hormone, Vitamine und Cytokine umfasst und einen pH-Wert zwischen 5-6 hat, unter Temperaturbedingungen, die jene des natürlichen Habitats von *D. antarctica* simulieren;
   e) Induzieren des Pflanzenmaterials zur Herstellung von sekundären Substanzen durch Anwenden von UV-B-Lichtimpulsen;

   wobei Folgende in Schritt d) verwendet werden: Zucker ausgewählt aus Saccharose, Glucose oder Fructose; Hormone ausgewählt aus Benzylaminopurin (BAP), Isopentenyladenin (2IP), Indolbuttersäure (IBA), GA3 oder ein Gemisch davon; Vitamine ausgewählt aus Biotin und Cytokinen; des Weiteren, in Schritt d), eine spezifische Nährstoffdosis auf das Pflanzenmaterial aufgetragen wird, während die Immersionszeit des Pflanzenmaterials in den Nährstoffen oder im Kulturmedium kontrolliert wird; und
   Schritt e) die Anwendung von 30 min UV-B-Licht alle 6 Stunden über einen Kultivierungszeitraum von 7-21 Tagen umfasst.

2. Verfahren für eine *In-vitro*-Kultur und Mikrovermehrung von *D. antarctica* nach Anspruch 1, wobei das Verfahren nach Schritt e) des Weiteren umfasst:

f) Bestimmen der Konzentration der gesamten Phenole, die durch das Pflanzenmaterial nach Schritt e) hergestellt wurden, und Bestimmen der Oxidationskapazität derselben; und/oder

g) Phenolextraktion und chemische Charakterisierung davon.

**3.** Verfahren für eine *In-vitro*-Kultur und Mikrovermehrung von *D. antarctica* nach Anspruch 1, wobei Schritt d) in einem Kulturmedium durchgeführt wird, das 1-3% Saccharose umfasst.

**4.** Verfahren für eine *In-vitro*-Kultur und Mikrovermehrung von *D. antarctica* nach Anspruch 1, wobei Schritt d) in einem Kulturmedium durchgeführt wird, das 0,2 mg/ml Kinetin umfasst.

**5.** Verfahren für eine *In-vitro*-Kultur und Mikrovermehrung von *D. antarctica* nach Anspruch 1, wobei Schritt d) in einem Kulturmedium durchgeführt wird, das 0,3 mg/ml BAP umfasst.

**6.** Verfahren für eine *In-vitro*-Kultur und Mikrovermehrung von *D. antarctica* nach Anspruch 1, wobei Schritt d) in einem Kulturmedium durchgeführt wird, das 0,1 mg/ml Biotin umfasst.

**7.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 2-5 g/l MS Basal Medium (*PhytoTech* Lab™), 20 g/l Saccharose, 0,2 mg/ml Kinetin, 0,3 mg/ml BAP, 0,1 mg/ml Biotin, 0,01 mg/ml IBA und 0,01 mg/ml GA3 umfasst.

**8.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 2-5 g/l MS Basal Medium (*PhytoTech* Lab™), 10-30 g/l Saccharose, 0,1-0,55 mg/ml 2IP umfasst.

**9.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 4,4 g/l MS Basal Medium (*PhytoTech* Lab™), 20 g/L Saccharose, 0,01 mg/ml IBA und 0,01 mg/ml GA3 umfasst.

**10.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 4,43 g/l MS Basal Medium (*PhytoTech* Lab™), 20 g/l Saccharose, 0,01 mg/ml IBA, 0,3 mg/ml BAP umfasst und einen pH-Wert zwischen 5,6-5,7 hat.

**11.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 4,43 g/l MS Basal Medium (*Phyto*Tech Lab™), 20 g/l Saccharose, 0,05 mg/ml IBA, 0,3 mg/ml BAP umfasst und einen pH-Wert zwischen 5,6-5,7 hat.

**12.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 4,43 g/l MS Basal Medium (*Phyto*Tech Lab™), 20 g/l Saccharose, 0,3 mg/ml BAP umfasst und einen pH-Wert zwischen 5,6-5,7 hat.

**13.** Verfahren für eine *In-vitro*-Kultur und Mikrovermehrung von *D. antarctica* nach Anspruch 1, wobei Schritt d) das Kultivieren und Mikrovermehren des Pflanzenmaterials in ein Kulturmedium umfasst, das 4,43 g/l MS Basal Medium (*Phyto*Tech Lab™), 20 g/l Saccharose, 0,02 mg/ml BAP umfasst.

**14.** Verfahren für eine *In-vitro*-Kultur und Mikrovermehrung von *D. antarctica* nach Anspruch 1, wobei das Verfahren des Weiteren Schritt d') des Hinzufügens chemischer Induktionsmittel durch Pulse, die ausgewählt sind aus Salzen, Metallen, organischen Substanzen wie beispielsweise Hormonen und dergleichen umfasst.

**15.** Verfahren für eine *In-vitro*-Kultur und Mikrovermehrung von *D. antarctica* nach Anspruch 1, wobei das Verfahren des Weiteren Schritt d") der Entnahme von Proben des Nährstoff- oder Kulturmediumverbrauchs durch das Pflanzenmaterial umfasst.

**Revendications**

1. Procédé de culture et de micropropagation en masse *in vitro* de *Descharnapsia antarctica (D. antarctica),* un matériel végétal, induisant en outre la génération ou la biosynthèse de métabolites antioxydants et photoprotecteurs caractéristiques dudit matériel végétal, comme le scopolétine, l'acide chlorogénique, le rutosine, et la quercétine, comprenant les étapes suivantes :

   a) recueillir le matériel végétal parent constitué de *Descharnapsia antarctica* ;
   b) récupérer ledit matériel végétal à partir du matériel végétal recueilli ;
   c) inoculer les racines dudit matériel végétal ;
   d) cultiver et micropropager *in vitro* ledit matériel végétal par immersion temporaire dans un milieu de base MS (*PhytoTech* Lab™) qui comprend en outre des sucres, des hormones, des vitamines et des cytokines et est à un pH entre 5 et 6, dans des conditions de température qui simulent celles de l'habitat naturel de *D. antarctica* ;
   e) induire la production de composés secondaires dans le matériel végétal en appliquant des impulsions de lumière UV-B,

   dans lequel les suivants sont utilisés à l'étape d) : des sucres sélectionnés parmi le saccharose, le glucose ou le fructose ; des hormones sélectionnées parmi la benzylaminopurine (BAP), l'isopentenyladénine (2iP), l'acide indole butyrique (AIB), le GA3 ou un mélange de ceux-ci ; des vitamines sélectionnées parmi la biotine et les cytokines ; en outre, à l'étape d), une dose spécifique de nutriments est fournie au matériel végétal, en contrôlant le temps d'immersion du matériel végétal dans les nutriments ou dans le milieu de culture et ;
   l'étape e) comprend l'application de lumière UV-B pendant 30 minutes toutes les 6 heures pour une période de culture de 7 à 21 jours.

2. Procédé de culture et de micropropagation *in vitro* de *D. antarctica* selon la revendication 1, dans lequel après l'étape e) il comprend en outre :

   f) la détermination de la concentration en composés phénoliques totaux produits par le matériel végétal après l'étape e), et la détermination de la capacité oxydante de ces derniers ; et/ou
   g) l'extraction des phénols et la caractérisation chimique de ceux-ci.

3. Procédé de culture et de micropropagation *in vitro* de *D. antarctica* selon la revendication 1, dans lequel l'étape d) est réalisée dans un milieu de culture comprenant 1 à 3 % de saccharose.

4. Procédé de culture et de micropropagation *in vitro* de *D. antarctica* selon la revendication 1, dans lequel l'étape d) est réalisée dans un milieu de culture comprenant 0,2 mg/mL de kinétine.

5. Procédé de culture et de micropropagation *in vitro* de *D. antarctica* selon la revendication 1, dans lequel l'étape d) est réalisée dans un milieu de culture comprenant 0,3 mg/mL de BAP.

6. Procédé de culture et de micropropagation *in vitro* de *D. antarctica* selon la revendication 1, dans lequel l'étape d) est réalisée dans un milieu de culture comprenant 0,1 mg/mL de biotine.

7. Procédé de culture et de micropropagation *in vitro* de *D. antarctica* selon la revendication 1, dans lequel l'étape d) comprend la culture et la micropropagation du matériel végétal dans un milieu de culture comprenant 2 et 5 g/L de milieu de base MS (*PhytoTech* Lab™), 20 g/L de saccharose, 0,2 mg/mL de kinétine, 0,3mg/mL de BAP, 0,1 mg/mL de biotine, 0,01 mg/mL d'AIB et 0,01 mg/mL de GA3.

8. Procédé de culture et de micropropagation *in vitro* de *D. antarctica* selon la revendication 1, dans lequel l'étape d) comprend la culture et la micropropagation du matériel végétal dans un milieu de culture comprenant 2 à 5 g/L de milieu de base MS (*PhytoTech* Lab™), 10 à 30 g/L de saccharose, 0,1 à 0,55 mg/mL de 2iP.

9. Procédé de culture et de micropropagation *in vitro* de *D. antarctica* selon la revendication 1, dans lequel l'étape d) comprend la culture et la micropropagation du matériel végétal dans un milieu de culture comprenant 4,4 g/L de milieu de base MS (*PhytoTech* Lab™), 20 g/L de saccharose, 0,01 mg/mL d'AIB et 0,01 mg/mL de GA3.

10. Procédé de culture et de micropropagation *in vitro* de *D. antarctica* selon la revendication 1, dans lequel l'étape d) comprend la culture et la micropropagation du matériel végétal dans un milieu de culture comprenant 4,43 g/L de milieu de base MS (*PhytoTech* Lab™), 20 g/L de saccharose, 0,01 mg/mL d'AIB, 0,3 mg/mL de BAP et à un pH entre 5,6 et 5,7.

11. Procédé de culture et de micropropagation *in vitro* de *D. antarctica* selon la revendication 1, dans lequel l'étape d) comprend la culture et la micropropagation du matériel végétal dans un milieu de culture comprenant 4,43 g/L de milieu de base MS (*PhytoTech* Lab™), 20 g/L de saccharose, 0,05 mg/mL d'AIB et 0,3 mg/mL de BAP et à un pH entre 5,6 et 5,7.

12. Procédé de culture et de micropropagation *in vitro* de *D. antarctica* selon la revendication 1, dans lequel l'étape d) comprend la culture et la micropropagation du matériel végétal dans un milieu de culture comprenant 4,43 g/L de milieu de base MS (*PhytoTech* Lab™), 20 g/L de saccharose, 0,3 mg/mL de BAP et à un pH entre 5,6 et 5,7.

13. Procédé de culture et de micropropagation *in vitro* de *D. antarctica* selon la revendication 1, dans lequel l'étape d) comprend la culture et la micropropagation du matériel végétal dans un milieu de culture comprenant 4,43 g/L de milieu de base MS (*PhytoTech* Lab™), 20 g/L de saccharose, 0,02 mg/mL de BAP.

14. Procédé de culture et de micropropagation *in vitro* de *D. antarctica* selon la revendication 1, comprenant en outre l'étape d') d'ajout, au moyen d'impulsions, d'inducteurs chimiques sélectionnés parmi des sels, des métaux, des composés organiques comme des hormones, etc.

15. Procédé de culture et de micropropagation *in vitro* de *D. antarctica* selon la revendication 1, comprenant en outre l'étape d") de prise d'échantillons de la consommation de nutriment ou du milieu de culture par le matériel végétal.

FIG. 1

EP 2 638 798 B1

FIG. 2

15

FIG. 3

## FIG. 4

## FIG 5

# FIG. 6

# FIG. 7

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Temporary Immersion Bioreactor: Engineering considerations and applications in plant micropropagation. **AFREEN, F.** Focus on Biotechnology-Ptant Tissue Culture Engineering. Springer, 2006, 187-200
- **AITKEN-CHRISTIE, J. ; SINGH A. ; HORGAN, K. ; THORPE, T.** Explant Developmental State and Shoot Formation in Pinus radiata Cotyledons. *Botanical Gazette,* 1985, vol. 146, 196-203
- **BANERJEE, S.** *In vitro multiplication of Centella asiatica, a medicinal herb from leaf explants,* 1999
- **CUBA, M. ; GUTIERREZ-MORAGA, A. ; BUTENDIEK, B ; GIDEKEL, M.** Micropropagation of Deschampsia antarctica - a frost resistant Antarctic plant. *Antarctic Sci.,* 2005, vol. 17, 69-70
- **ENDRESS, T.** Plant Cell Biotechnology. Springer-Verlag, 1994, 121-246
- **ETIENNE H ; BERTHOULY M.** Temporary immersion systems in plant micropropagation. *Plant Cell, Tissue and Organ Culture,* 2002, vol. 69, 215-231
- **ETIENNE, H. ; DECHAMP, E. ; BARRY-ETIENNE, D. ; BERTRAND, B.** Bioreactors in coffee micropropagation. *Brazilian Journal of Plant Physiology,* 2006, vol. 18, 45-54
- **MCALISTER, B. ; FINNIE, J. ; WATT, M. P ; BLAKEWAY, F.** Use of the temporary immersion bioreactor system (RITA) for production of commercial Eucalyptus clones in Mondi Forests (SA). *Plant Cell, Tissue and Organ Culture,* 2005, vol. 81, 347-358
- **PAEK K. ; HAHN E ; SON S.** Application of bioreactors for large-scale micro propagation system of plants. *In vitro Cell. Dev. Biol. - Plant,* 2001, vol. 37, 149-157
- **QUIALA, E. ; BARBÓN, R. ; JIMÉNEZ, E. ; DE FERIA, M. ; CHÁVEZ, M. ; CAPOTE, A. ; PÉREZ, N.** Biomass production of Cymbopogon citratus (DC) Stapf., a medicinal plant, in temporary immersion systems. *In Vitro Cellular & Developmental Biology,* 2006, vol. 42, 298-300
- **ROELS S. ; NOCEDA C. ; ESCALONA M. ; SANDOVAL J. ; CANAL M.J. ; RODRIGUEZ R. ; DEBERGH P.** The effect of headspace renewal in a Temporary Immersion Bioreactor of plantain (Musa AAB) shoot proliferation and quality. *Plant Cell, Tissue and Organ Culture,* 2006, vol. 84, 155-163

- **ZHU L. ; LI X. ; WELANDER M.** Optimization of growing conditions for the apple rootstock M26 grown in RITA containers using temporary immersion principle. *Plant Cell, Tissue and Organ Culture,* 2005, vol. 81, 313-318
- **ENDRESS, R.** Plant Cell Biotechnology. Springer-Verlag, 1994, 121-246
- **ETIENNE H ; BERTHOUIY. M.** Temporary immersion systems in plant micropropagation. *Plant Cell, Tissue and Organ Culture,* 2002, vol. 69, 215-231
- **ETIENNE H. ; BERTHOULY M.** Temporary immersion systems in plant micro propagation. *Plant Cell, Tissue and Organ Culture,* 2002, vol. 69, 215-231
- **ETIENNE, H. ; DECHAMP, E. ; BARRY-ETIENNE, D ; BERTRAND, B.** Bioreactors in coffee micropropagation. *Brazilian Journal of Plant Physiology,* 2006, vol. 18, 45-54
- **AFREEN, F.** Temporary Immersion Bioreactor: Engineering considerations and applications in plant micropropagation. *Focus on Biotechnology - Plant Tissue Culture Engineering,* 2006, 187-200
- **ROELS S. ; NOCEDA C. ; ESCALONA M. ; SARIDOVAL J. ; CANAL M.J. ; RODRIGUEZ R ; DEBERGH P.** The effect of headspace renewal in a Temporary Immersion Bioreactor of plantain (Musa AAB) shoot proliferation and quality. *Plant Cell, Tissue and Organ Culture,* 2006, vol. 84, 155-163
- **PAEK K. ; HAHN E ; SON S.** Application of bioreactors for large scale micropropagation system of plants. *In vitro Cell. Dev. Biol. - Plant,* 2001, vol. 37, 149-157
- **AFREEN, F.** Temporary Immersion Bioreactor: Engineering considerations and applications in plant micropropagation. *Focus on Biotechnology - Plant Tissue Culture Engineering,* 2006, 187-200
- **AITKEN-CHRISTIE, J. ; SINGH A. ; HORGAN, K. ; THORPE ; T.** Explant Developmental State and Shoot Formation in Pinus radiata Cotyledons. *Botanical Gazette,* 1985, vol. 146, 196-203
- **MCALISTER, B. ; FINNIE, J. ; WATT, M. P ; BLAKEWAY, F.** Use of the temporary immersion bioreactor system (RITA) for production of commercial Eucalyptus clones in Mondi Forests (SA). *Plant Cell, Tissue and Organ Culture,* 2005, vol. 81, 347-358

- **ZHU L. ; LI X. ; WELANDER M.** Optimization of growing conditions for the apple rootstock M26 grown in RITA containers using temporary immersion principle. *Plant Cell, Tissue and Organ Culture,* 2005, vol. 81, 313-318
- **ROELS S. ; NOCEDA C. ; ESCALONA M. ; SANDOVAL J. ; CANAL M.J. ; RODRIGUEZ R. ; DEBERGH P.** The effect of headspace renewal in a Temporary Immersion. Bioreactor of plantain (Musa AAB) shoot proliferation and quality. *Plant Cell, Tissue and Organ Culture,* 2006, vol. 84, 155-163
- **ETIENNE, H. ; DECHAMP, E. ; BARRY-ETIENNE, D ; BERTRAND, B.** Bioreactors in coffee micropropagation. *Brazilian Journal of Plant Physiology,* 2006, vol. 18, 45-54
- **ROELS S. ; NOCEDA C. ; ESCALONA M. ; SANDOVAL J. ; CANAL M.J. ; RODRIGUEZ R ; DEBERGH P.** The effect of headspace renewal in a Temporary Immersion Bioreactor of plantain (Musa AAB) shoot proliferation and quality. *Plant Cell, Tissue and Organ Culture,* 2006, vol. 84, 155-163
- **BANERJEE, S.** *In vitro multiplication of Centella asiatica, a medicinal herb from leaf explants,* 1999
- **CUBA, M. ; GUTIERREZ-MORAGA, A. ; BUTENDIEK, B. ; GIDEKEL, M.** Micropropagation of Deschampsia antarctica - a frost-resistant Antarctic plant. *Antarctic Sci.,* 2005, vol. 17, 69-70
- **ENDRESS, R.** Plant Cell Biotechnology. Springer-Verlag, 1994, 121-246
- **MURASHIGE T. ; SKOOG F.** A revised medium for rapid growth and bioassays with tobacco tissue cultures. *Physiol. Plant,* 1962, vol. 15, 473-497
- **ROELS S. ; NOCEDA C. ; ESCALONA M. ; SANDOVAL J. ; CANAL M.J. ; RODRIGUEZ R ; DEBERGH P.** The effect of headspace renewal in a Temporary Immersion Bioreactor of plantain (Musa AAB) shoot proliferation and quality. *Plant Cell, Tissue and Organ Culture,* 2006, vol. 84, 155-163